# EUROPEAN PATENT APPLICATION

(11) **EP 3 779 435 A1**
(43) Date of publication of application: **17.02.2021**
(21) Application number: 19792300.6
(22) Date of filing: 19.02.2019
(51) Int. Cl.: G01N 33/50, B01L 3/00

(54) **MICROFLUIDIC CHIP AND ANALYTICAL INSTRUMENT PROVIDED WITH MICROFLUIDIC CHIP**

(30) Priority: 27.04.2018 CN 201820621664 U
(71) Applicant: Guangzhou Wondfo Biotech. Co., Ltd., Guangdong 510663 (CN)
(72) Inventor: MENG, Xuan, Guangdong 510663 (CN); WAN, Huifang, Guangdong 510663 (CN); HU, Haisheng, Guangdong 510663 (CN); LI, Wenmei, Guangdong 510663 (CN)
(74) Representative: Meyer, Thorsten
(86) International application number: PCT/CN2019/075460
(87) International publication number: WO 2019/205780

(57) **Abstract**

The present invention discloses a micro-fluidic chip, comprising a chip main body, a sample inlet, a liquid driving force inlet, a main fluid channel and multiple functional chambers provided on the chip main body. The micro-fluidic chip of the present invention identifies, positions and quantifies a liquid by means of a specific liquid quantification chamber, thereby decreasing chip manufacturing process difficulty, and increasing quantification accuracy.

## Description

### FIELD OF THE INVENTION

The present invention relates to the technical field of medical equipment, in particular to a micro-fluidic chip and analytical instrument provided with the micro-fluidic chip.

### BACKGROUND OF THE INVENTION

In Vitro Diagnosis (IVD) refers to taking samples (blood, body fluids, tissues, etc.) from the human body for detection and analysis to diagnose diseases. The detection process requires corresponding instruments and reagents, and these instruments and reagents constitute In Vitro Diagnosis system. In Vitro Diagnosis systems are generally divided into two types: one is represented by the testing center laboratory, which has modular, automated, and streamlined systems for sample testing, thus has advantages of high throughput, high efficiency, and high sensitivity, but the entire system also has the disadvantages that it is expensive, occupies a large volume, and requires professionals to operate it, and it is mainly used in large scale hospitals; the other is represented by "point-of-care testing (POCT)", its system has the characteristics of integration, miniaturization, and the ability to conduct sample inspection anytime and anywhere, so it also has the advantages of affordable price, simple operation, and timely results report, but compared with the testing center laboratory, its test results still have the shortcomings of low sensitivity and low stability.

For POCT, micro-fluidic technology is applied to *in vitro* diagnostic products both domestically and abroad. Microfluidics is an interdisciplinary subject that controls and operates microfluids on a chip provided with microchannels, involving biology, chemistry, fluid physics, electronics, optics, mechanical engineering and other fields. Micro-fluidic devices are usually called "micro-fluidic chips", also known as "Lab on a Chip". Usually, the basic operations such as sample preparation, reaction, separation, and detection of biological, chemical, and medical analysis processes are concentrated on a chip to carry out systematic functions. The existing micro-fluidic chips mainly focus on qualitative detection, and the quantitative-detection-oriented micro-fluidic chips are rare, and the existing manufacturing of quantitative micro-fluidic chips are complicated and low efficiency. For example, the Chinese patent application with publication number "CN105214744A" discloses "A magnetic particle chemiluminescence micro-fluidic chip", the micro-fluidic chip comprises a top plate and a bottom plate, wherein the top plate comprises an air pump, a sample inlet, a sample filling area, a marking ligand storage pool and a sample mixing area; the bottom plate comprises a filter area, a magnetic particle coating area, a washing area, a detecting area, a washing liquid storage pool, a light-emitting substrate storage pool and a liquid release channel; the top plate and the bottom plate both comprise liquid sensing devices, to determine the flowing state of the liquid in the micro-fluidic chip and whether bubbles are mixed into the micro-fluidic chip. The chip in this patent application employs a multi-layer structure, and it uses holding bags with a specific volume to achieve liquid quantification. Although this quantitative structure is simple, it is very prone for the liquid to remain on the inner surface of the bag (that is, when the liquid is pressed out of the holding bag, part of the liquid is hung on the inner surface of the bag, and it is not guaranteed to press out all the liquid), and the amount of deformation of the holding bag when it is squeezed is not the same every time, so each time the amount of liquid remaining in the holding bag is inconsistent, and the amount of liquid squeezed out is different, especially when requiring a small amount of liquid, the error from the holding bag is even bigger. With regard to micro-fluidic chip, all that quantity needed is tens of microliters, so the quantitative accuracy of such holding bag cannot meet the requirements, and the quantitative accuracy is poor, which affects the detection result,also the holding bag needs to be built into the chip, which increases the manufacturing difficulty.

### SUMMARY OF THE INVENTION

To solve the above-mentioned problems, on the one hand, the present invention provides a micro-fluidic chip, which can realize quantitative detection and has a simple structure, thus reduces the manufacturing difficulty of the chip.

The technical solution adopted by the present invention is: a micro-fluidic chip, comprising a chip main body, and a sample inlet, a liquid driving force inlet, a main fluid channel and multiple functional chambers arranged on the chip main body;

The main fluid channel communicates with the multiple functional chambers, the sample inlet and the liquid driving force inlet respectively communicate with the main fluid channel, and the liquid driving force inlet is used to connect the liquid driving device to drive liquid into or out of the functional chambers;

At least one of the multiple functional chambers is a liquid quantification chamber; the liquid quantification chamber has a predetermined volume, and a liquid identification site is provided at the liquid outlet of the liquid quantification chamber, the liquid to be quantitatively determined flows into the liquid quantification chamber from the liquid inlet of the liquid quantification chamber, fills the liquid quantification chamber and then reaches the liquid outlet.

In one of the embodiments, the liquid quantification chamber includes a reagent quantification chamber, the liquid inlet of the reagent quantification chamber communicates with one end of the reagent subchannel, and the other end of the reagent subchannel communicates with the reagent inlet.

In one of the embodiments, a liquid identification site is also provided at the liquid inlet of the liquid quantification chamber.

In one of the embodiments, the liquid driving device is a plunger pump.

In one of the embodiments, the liquid quantification chamber further includes a sample quantification chamber, and the liquid sample flows into the sample quantification chamber through the sample inlet for quantification; the sample quantification chamber is located upstream of the reagent quantification chamber;

The micro-fluidic chip is also provided with an air inlet and an air subchannel communicating with the air inlet. One end of the air subchannel communicates with the air inlet, the other end communicates with the main fluid channel between the sample quantification chamber and the sample inlet, and the junction point of the other end of the air subchannel and the main fluid channel is adjacent to the sample quantification chamber.

In one of the embodiments, the functional chambers include a detection chamber, the detection chamber has a predetermined volume, and a liquid identification site is provided at the liquid outlet of the detection chamber, and the liquid to be detected flows into the detection chamber through the liquid inlet of the detection chamber, fills the detection chamber and reaches the liquid outlet.

In one of the embodiments, a liquid identification site is also provided at the liquid inlet of the detection chamber.

In one of the embodiments, the liquid identification site is used to locate the liquid identification device; the liquid identification device includes a light source generating module and a photoelectric sensor;

The liquid identification site includes an upper site for locating the light source generating module and a lower site for locating the photoelectric sensor, the upper site and the lower site are respectively provided outside the chip main body, the positions of the upper site and the lower site correspond to the corresponding liquid outlet or liquid inlet, so that the positioned light source generating module, the corresponding liquid outlet or liquid inlet, and the photoelectric sensor are successively arranged in a vertical line.

In one of the embodiments, the liquid quantification chamber is a chamber having a hexagonal structure.

In one of the embodiments, the width of the liquid inlet of the liquid quantification chamber is 0.3-3mm and the height is 0.3-3mm; the width of the liquid outlet of the liquid quantification chamber is 0.3-3mm, and the height is 0.3 -3mm; or

The surface of the liquid quantification chamber is a surface formed by hydrophilic surface modification; the width of the liquid inlet of the liquid quantification chamber is 0.3-5 mm, and the height is 0.3-3 mm; the width of the liquid outlet of the liquid quantification chamber is 0.3-5mm, and the height is 0.3-3mm; or

The surface of the liquid quantification chamber is a surface formed by hydrophobic surface modification, the width of the liquid inlet of the liquid quantification chamber is 0.3-2 mm and the height is 0.3-3 mm; the width of the liquid outlet of the liquid quantification chamber is 0.3-2mm, and the height is 0.3-3mm.

In one of the embodiments, the chip main body includes a top plate and a bottom plate; the top plate and the bottom plate are stacked and connected, the main fluid channel and the multiple functional chambers are provided at the connecting point of the top plate and the bottom plate.

In one of the embodiments, the bottom plate is a smooth flat plate, and the top plate is provided with micro pores, micro channels or micro cavities, to form the sample inlet, the liquid driving force inlet, the main fluid channel or functional chambers together with the bottom plate.

In one of the embodiments, the sample inlet and the liquid driving force inlet are respectively provided at two ends of the main fluid channel.

On the other hand, the present invention also provides an analytical instrument provided with a micro-fluidic chip, which includes an instrument frame, at least one reagent storage pool, a liquid driving device, a detection device, and the above-mentioned micro-fluidic chip; wherein the micro-fluidic chip is installed in the instrument frame; the liquid driving device is connected to the liquid driving force inlet of the micro-fluidic chip; the reagent storage pool and the corresponding reagent inlet can communicate on and off with each other; the detection device is used for receiving and processing the detection signal sent by the micro-fluidic chip.

In one of the embodiments, the liquid driving device is a plunger pump; each of the reagent storage pools is provided with an opening communicating the outside air.

Comparing with the prior art, the present invention has the following beneficial effects:

The micro-fluidic chip provided by the present invention realizes the quantification of liquid by a specific liquid quantification chamber together with a liquid driving device. Comparing with the existing technology of realizing quantification by squeezing the reagent package embedded in the chip, the liquid quantification chamber of the present invention improves the accuracy of quantification; and the reagents can be externally placed on the chip. Compared with the multi-layer chip combination and the reagent package embedded in the chip in the prior art, the difficulty of the chip manufacturing process is reduced, and the detection accuracy is improved.

The chip main body of the micro-fluidic chip of the present invention can include a top plate and a bottom plate that are stacked, and the top plate can be provided for any structure that needs to be processed. The bottom plate is only a smooth flat plate, which can further reduce the difficulty of the chip manufacturing process and improve production effectiveness.

### BRIEF DESCRIPTION OF THE DRAWINGS

**FIG. 1** is a structural schematic diagram of an embodiment of a micro-fluidic chip provided by the present invention;
**FIG. 2** is a schematic cross-sectional diagram of the liquid identification device provided by the present invention;
**FIG. 3** is a structural diagram of the photoelectric sensors arrangement of an embodiment of the micro-fluidic chip provided by the present invention;
**FIG. 4** is a schematic cross-sectional diagram of the position of the magnet when the micro-fluidic chip provided by the present invention is used;
**FIG. 5** is a schematic structural diagram of an embodiment of the liquid driving device provided by the present invention;

Wherein, reference signs are as follows: 1. top plate; 2. sample inlet; 3. whole blood filtration area; 4. sample quantification area; 5. enzyme-labeled primary antibody embedding area; 6. first mixing channel; 7. magnetic-labeled secondary antibody embedding area; 8. second mixing channel; 9. chemiluminescence detection area; 10. diluent inlet; 11. luminescent substrate liquidinlet; 12. washing liquid inlet; 13. liquid driving force inlet; 14. air inlet 15. sealing gasket; 16. diluent subchannel; 17.luminescent substrate liquid subchannel; 18. washing liquid subchannel; 19. plunger pump; 20. bottom plate; 21. diluent storage pool; 22. luminescent substrate liquid storage pool; 23.washing liquid storage pool; 24. waste liquid pool; 25a/25b. magnet; 26. magnetic beads; 27. air subchannel; 28.light resource generating module; 29. photoelectric sensor; 191. liquid inlet of the plunger pump; 192, liquid outlet of the plunger pump; 193. plunger; 194. pump chamber.

### DETAILED DESCRIPTION OF THE INVENTION

The following describes the technical solutions in the embodiments of the present invention clearly and completely in conjunction with the accompanying drawings in the embodiments of the present invention. Obviously, the described embodiments are only a part of the embodiments of the present invention, rather than all the embodiments. Based on the embodiments of the present invention, all other embodiments obtained by those of ordinary skill in the art without creative work shall fall within the protection scope of the present invention.

### Example 1

This embodiment provides a micro-fluidic chip. The micro-fluidic chip includes a chip main body, and a sample inlet, a liquid driving force inlet, a main fluid channel, and multiple functional chambers provided on the chip main body. The detailed description is given below.

In this embodiment, the main fluid channel communicates with multiple functional chambers to guide the flow of fluid between the functional chambers.

In this embodiment, the functional chambers at least have the function of accommodating. Preferably, the functional chambers have other functions in addition to the accommodating function. Other functions can be implemented in the functional chambers or combined with the with the external necessary components of the functional chambers(these necessary components can be fixed outside the chip without being provided inside the chip or on the surface of the chip).

In this embodiment, the sample inlet and the liquid driving force inlet respectively communicate with the main fluid channel, the liquid driving force inlet is used to connect the liquid driving device to drive the liquid into or out of the functional chambers, and the sample inlet is used to introduce the liquid sample into the main fluid channel, the liquid sample enters each functional chamber through the main fluid channel.

In this embodiment, at least one of the multiple functional chambers is a liquid quantification chamber; the liquid quantification chamber has a predetermined volume, and a liquid identification site is provided at the liquid outlet of the liquid quantification chamber. The liquid inlet of the liquid quantification chamber flows into the liquid quantification chamber, fills the liquid quantification chamber and then reaches the liquid outlet. The liquid identification siteis used to locate or fix the liquid identification device, and the liquid identification device is used to identify the liquid. When the liquid flows to the liquid identification site, the liquid identification device can identify the liquid and provide a liquid arrival signal; therefore, when the liquid reaches the liquid outlet, the liquid identification device can provide a liquid arrival signal indicating that the liquid has filled the liquid quantification chamber. At this time, the liquid driving device is controlled to stop driving the liquid, to realize the quantification of liquid in the liquid quantification chamber.

The micro-fluidic chip provided in this embodiment realizes the quantification of liquid by a specific liquid quantification chamber together with a liquid driving device. Comparing with the existing technology of realizing quantification by squeezing the reagent pack embedded in the chip, the liquid quantification chamber of the present invention improves the accuracy of quantification. The reagent can be placed outside the chip, comparing with the multi-layer chip combination and the reagent package embedded in the chip in the prior art, the difficulty of the manufacturing process of the chip is reduced and the detection accuracy is improved.

It should be declared that the main fluid channel and the multiple functional chambers can be formed inside the chip main body by laser processing, mold injection processing, etc., or the top plate and the bottom plate can also be set separately, and particular structures can be made on the top plate or the bottom plate, and then the top plate or the bottom plate can be assembled together. Since the former processing method is comparably complicated, in a preferred embodiment, the chip main body includes a top plate and a bottom plate; the top plate and the bottom plate are stacked and connected; at the connection place of the top plate and the bottom plate there provided with the main fluid channel and the multiple functional chambers; more preferably, the bottom plate is a smooth flat plate, and the top plate is provided with micro pores, micro channels or micro cavities to form the sample inlet, the liquid driving force inlet, the main fluid channel or the functional chambers together with the bottom plate. Such micro-fluidic chips are more convenient to prepare, the difficulty of the production process is reduced, and only specific structures on the top plate need to be processed, which improves production efficiency. Specifically, the bottom plate is a smooth flat plate, the top plate is provided with multiple micro channels to form the main fluid channel together with the bottom plate, and the top plate is provided with multiple micro cavities to form the multiple functional chambers together with the bottom plate, and the top plate is provided with multiple holes to form the sample inlet or liquid driving force inlet together with the bottom plate; in order to facilitate sampling, the size of the sample inlet is usually larger than the size of other inlets.

The number of liquid quantification chambers, the types of liquids (such as liquid samples, reaction reagents, sample processing reagents, etc.) that the liquid quantification chambers quantified, setting positions, and types of other functional chambers can be selected according to actual needs.

Preferably, the liquid quantification chamber includes a reagent quantification chamber. The liquid inlet of the reagent quantification chamber communicates with one end of the reagent subchannel, and the other end of the reagent subchannel communicates with the reagent inlet. The reagent enters the reagent quantification chamber through the reagent inlet and the reagent subchannel for quantification. When the micro-fluidic chip is used for quantitative detection, the amount of liquid samples (i.e., the liquid to be tested flowing from the sample inlet) and reagents (e.g. reaction reagents, sample processing reagents, etc.) needs to be quantified. Usually, the quantification of reagents needs to be conducted inside the chip, the liquid sample can optionally be quantified outside the micro-fluidic chip. In this embodiment, the reagent quantification chamber is used for reagent quantification. Here, "the liquid quantification chamber includes the reagent quantification chamber" should be understood as "the liquid quantification chamber should at least have a type of reagent quantification chamber for quantifying reagents", certainly, it can also further include liquid quantification chamber for quantifying other liquid types, such as the sample quantification chamber for quantifying liquid samples, etc.

The number of reagent quantification chambers can be one, two or more, which can be selected according to the actual needs of the micro-fluidic chip. For example, when the micro-fluidic chip is a chemiluminescence micro-fluidic chip, in order to achieve chemiluminescence quantitative detection, at least one reagent quantification chamber should be provided to quantify the luminescent substrate liquid, and the remaining reaction materials such as the enzyme-labeled primary antibody, the magnetic-labeled secondary antibody can be embedded in the two functional chambers i.e. the enzyme-labeled primary antibody embedding chamber and the magnetic-labeled secondary antibody embedding chamber; preferably, the magnetic-labeled secondary antibody embedding chamber is the reagent quantification chamber, inside which not only embedded the magnetic-labeled secondary antibody, but also be used to quantify the luminescent substrate liquid; more preferably, the magnetic-labeled secondary antibody embedding chamber is also used for magnetic bead washing.

Optionally, the reagent inlet and the reagent storage pool can be connected on and off by a valve, the reagent storage pool is provided with an opening that communicates with the outside air, and the reagent storage pool is provided with an opening to facilitate the liquid driving device to introduce the liquid into the chip. In order to facilitate the preparation of the chip, preferably, the reagent storage pool is provided outside the micro-fluidic chip, and when using it, the reagent storage pool is installed at the place of the reagent inlet to introduce the reagent into the chip.

Optionally, a liquid identification site is also provided at the liquid inlet of the liquid quantification chamber. This liquid identification site can also be used to locate or fix the liquid identification device. This can facilitate the monitoring and control of the liquid flow in the chip, and also realize the mixing of two quantification liquids, such as the liquid sample and the reagent. Inside the chip, if the two liquids are to be mixed, they need to be in contact with no gaps between each other. But if the micro-fluidic chip of the present invention is to realize the quantification of the liquid and the contact of the two liquids at the same time, it requires the quantified liquid stays at a predetermined position, and the another liquid should preferably start to flow into the liquid quantification chamber from this predetermined position, and being quantified in the liquid quantification chamber. The best choice for this predetermined position is the liquid inlet of the liquid quantification chamber: setting the liquid identification site at the liquid inlet to locate the liquid identification device, which can provide the stay indication signal of one of the liquids and the feeding signal of the other liquid, with the cooperation of the liquid identification device of the liquid outlet of the liquid quantification chamber, the quantification of the liquid and the contact of the two liquids can be realized. Next, taking the mixing of liquid samples and reagents as an example to illustrate a method of using the micro-fluidic chip:

When the micro-fluidic chip is used, the reagent inlet and the reagent storage pool can be connected on and off by a valve, and the reagent storage pool is provided with an opening communicating with the outside air. When the liquid sample (in order to facilitate accurate mixing or reaction with a quantification reagent, the liquid sample is preferably a predetermined amount of liquid sample) is driven by the liquid driving device to flow from the sample inlet to the liquid inlet of the reagent quantification chamber through the main fluid channel, the liquid identification device located at the liquid identification site of the reagent quantification chamber obtains the signal and controls the liquid driving device to stop its driving action. At this time, the liquid sample stops flowing, and the front end of the liquid sample stays at the liquid inlet; then, close the air inlet and open the valve between the reagent inlet and the reagent storage pool. Under the action of the liquid driving device, the reagent enters the reagent quantification chamber from the reagent inlet through the reagent subchannel and the liquid inlet of the reagent quantification chamber. When it flows to the liquid outlet of the reagent quantification chamber, the reagent fills the reagent quantification chamber. At this time, close the valve between the reagent inlet and the reagent storage pool, and open the air inlet. The reagent and liquid sample quantified by the reagent quantification chamber continue to flow under the action of the liquid driving device, and can be under the positive pressure of the liquid driving device. The mixing and/or reaction can be realized under alternating positive pressure and negative pressure of the liquid driving device.

In order to reduce the influence of the reagent subchannel on the liquid identification device at the reagent quantification chamber liquid inlet and to facilitate processing and manufacture, specifically, one end of the reagent subchannel communicates with the liquid inlet of the reagent quantification chamber through the main fluid channel. The junction point of the reagent subchannel and the main fluid channel is adjacent to the liquid inlet of the reagent quantification chamber, so that the quantification is within a controllable range and the quantification error is reduced. For example, the distance between the junction point of the reagent subchannel and the main fluid channel and the liquid inlet of the reagent quantification chamber is 0.5-10mm (preferably 0.5-2mm).

Optionally, the sample inlet and the liquid driving force inlet are respectively arranged at both ends of the main fluid channel.

Optionally, the functional chambers includes a test chamber, the test chamber has a predetermined volume, and a liquid identification site is provided at the liquid outlet of the test chamber, the liquid to be detected flows into the test chamber and reaches the liquid outlet after filling the test chamber. The liquid identification site provided at the liquid outlet of the test chamber can be used to locate or fix the liquid identification device. When the liquid to be detected reaches the liquid outlet of the test chamber, the liquid identification device sends a signal, and the liquid driving device controls the liquid to be detected to stop flowing, at this time the test can be performed. Further, there is also a liquid identification site at the liquid inlet of the test chamber.

Optionally, the liquid quantification chamber further includes a sample quantification chamber, and the liquid sample flows into the sample quantification chamber through the sample inlet for quantification; the sample quantification chamber is located upstream of the reagent quantification chamber; the micro-fluidic chip is also provided with an air inlet, one end of the air subchannel communicates with the air inlet, and the other end communicates with the main fluid channel between the sample quantification chamber and the sample inlet, and the junction point of the other end of the air subchannel and the main fluid channel is adjacent to the sample quantification chamber; here, "adjacent" can generally be understood as "0.5-10 mm (preferably 0.5-2 mm) from the liquid inlet of the sample quantification chamber".

When the micro-fluidic chip is used, the air inlet and the air pipe outside the chip can be connected on and off by a valve to control the air entering the chip. The liquid sample flows into the sample quantification chamber from the liquid inlet of the sample quantification chamber through the sample inlet under the action of the liquid driving device. When the liquid sample flows to the liquid outlet of the sample quantification chamber, the sample quantification chamber is filled. The liquid identification device located on the liquid identification site of the liquid outlet sends out an indication signal to control the air inlet to be opened. Since the air flow in the air subchannel requires a small driving force, the liquid sample flow requires a greater driving force, therefore, the liquid sample stops at the junction point of the air subchannel and the main fluid channel and does not continue to flow into the sample quantification chamber. Thus, the quantification of the liquid sample in the sample quantification chamber can be completed. The quantified liquid sample can continue to flow to the liquid inlet of the reagent quantification chamber under the action of the liquid driving device. After the reagent quantification chamber completes the quantification of the reagent (the process is as described above), the liquid sample and reagent are mixed and/or reacted under alternating positive and negative pressures of the liquid actuators.

The micro-fluidic chip in this embodiment is provided with a sample quantification chamber, which facilitates the quantification of liquid samples without requiring additional quantification outside the chip, making the chip more convenient to use.

Optionally, the liquid sample is whole blood, a whole blood filtration chamber is provided between the sample inlet and the sample quantification chamber, and a whole blood filtration membrane is provided in the whole blood filtration chamber; when the micro-fluidic chip is used in clinical diagnosis, whole blood is a common test sample, and it is usually necessary to perform whole blood separation to separate the serum or plasma from the whole blood, and then react with reagents; a whole blood filtration chamber is provided in the chip, thus facilitating the detection. Compared to the method of first quantifying whole blood and then performing whole blood separation, a whole blood filtration chamber is provided between the sample inlet and the sample quantification chamber , and the amount of serum or plasma can be directly quantified by the sample quantification chamber , and the measurement results are more accurate. The material of the whole blood filtration membrane can be glass fiber, cotton linter fiber, polyester fiber, or blend fiber; optionally, the thickness of the whole blood filtration pad is 0.2-2.5mm; the adsorption speed of the whole blood filtration pad is 4-150 s/4cm, and the water absorption is 30-250 mg/cm².

Optionally, the liquid outlet of the whole blood filtration area is a triangular liquid outlet; the whole blood filtration area has an area of 30-300mm², a width of 2-20mm, a length of 5-25mm, a depth of 0.3-3mm, and the angle of the front triangle is 15 -160°.

### Example 2

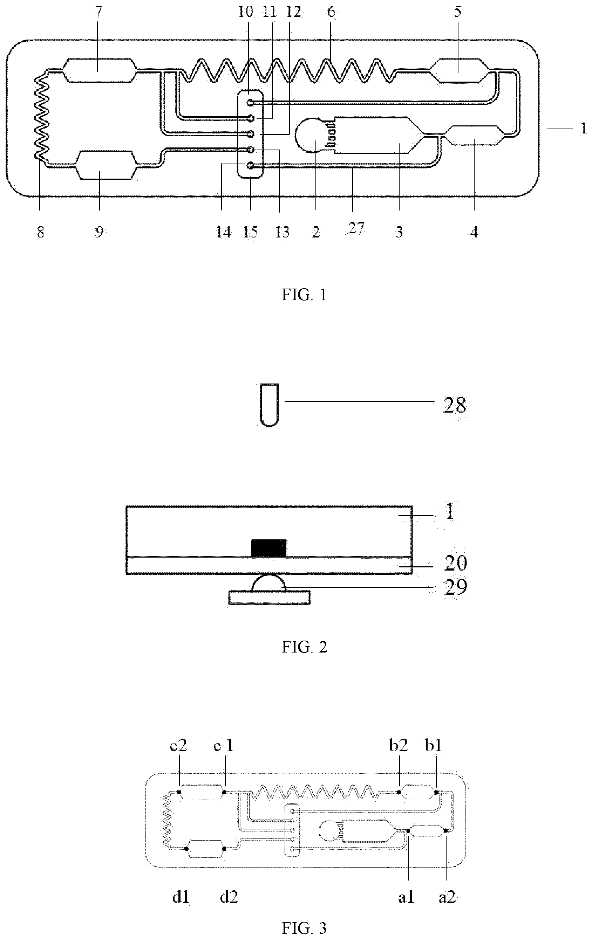

Refer to **FIG. 1** to **FIG. 5****,** this embodiment provides a chemiluminescence micro-fluidic chip, which includes a chip main body, and Sample Inlet2, Liquid Driving Force Inlet13, Luminescent Substrate Liquid Inlet 11, Washing Liquid Inlet 12, Luminescent Substrate Liquid Subchannel 17, Washing Liquid Subchannel 18, main fluid channel and multiple functional chambers; the detailed description will be given below.

In this embodiment, the main fluid channel communicates with multiple functional chambers to guide fluid flow between the functional chambers.

The functional chambers include an Enzyme-labeled Primary Antibody Embedding Area 5, a Magnetic Beads-labeled Secondary Antibody Embedding Area 7 and a Chemiluminescence Detection Area 9 which are successively connected by the main fluid channel.

Wherein, the enzyme-labeled primary antibody is embedded in Enzyme-labeled Primary Antibody Embedding Area 5; the magnetic-labeled secondary antibody is embedded in Magnetic Beads-labeled Secondary Antibody Embedding Area 7; Magnetic Beads-labeled Secondary Antibody Embedding Area 7 is the liquid quantification chamber; the liquid quantification chamber is used to quantify liquid. After the liquid to be quantified (e.g. luminescent substrate liquid) enters the liquid quantification chamber, it can be quantified in the liquid quantification chamber (that is, the required amount of liquid is obtained), to react with the quantitative liquid sample Or other reaction reagents to achieve quantitative detection.

In this embodiment, the liquid quantification chamber has a predetermined volume, and a liquid identification site is provided at the liquid outlet of the liquid quantification chamber. The liquid to be quantified flows from the liquid inlet of the liquid quantification chamber into the liquid quantification chamber, and arrives the liquid outlet after being filled with the liquid quantification chamber; the liquid identification site is used to locate or fix the liquid identification device, and the liquid identification device is used to identify the liquid. When the liquid reaches the liquid outlet, the liquid identification device can provide a liquid arrival signal indicating that the liquid has filled the liquid quantification chamber. At this time, the liquid driving device is controlled to stop driving the liquid, and the quantification of the liquid in the liquid quantification chamber can be realized. The chemiluminescence micro-fluidic chip realizes the quantification of liquid by a specific liquid quantification chamber combined with a liquid driving device, which can improve the accuracy of quantification.

In this embodiment, Chemiluminescence Detection Area 9 is used to accommodate the chemiluminescence reaction product to complete the detection process in combination with an external detection device.

Sample Inlet 2 and Liquid Driving Force Inlet 13 respectively communicate with the main fluid channel. Liquid Driving Force Inlet 13 is used to connect the liquid driving device to drive the liquid into or out of the functional chambers; Sample Inlet 2 is used to introduce the liquid sample into the main fluid channel, The liquid sample enters each functional chamber through the main fluid channel.

In this embodiment, one end of Luminescent Substrate Liquid Subchannel 17 communicates with Luminescent Substrate Liquid Inlet 11, and the other end communicates with the liquid inlet of Magnetic Beads-labeled Secondary Antibody Embedding Area 7. The luminescent substrate liquid passes through Luminescent Substrate Liquid Inlet 11 and Luminescent Substrate Liquid Inlet 11. Liquid Subchannel 17 enters Magnetic Beads-labeled Secondary Antibody Embedding Area 7 for quantification.

One end of Washing Liquid Subchannel 18 communicates with Washing Liquid Inlet 12, and the other end communicates with the liquid inlet of Magnetic Beads-labeled Secondary Antibody Embedding Area 7. The washing liquid enters Magnetic Beads-labeled Secondary Antibody Embedding Area 7 through Washing Liquid Inlet 12 and Washing Liquid Subchannel 18 to perform magnetic bead washing.

When the micro-fluidic chip of this embodiment is used, Luminescent Substrate Liquid Inlet 11 and Washing Liquid Inlet 12 are respectively connected on and off to Luminescent Substrate Liquid Storage Pool 22 and Washing Liquid Storage Pool 23 by Valves V2 and V3. Luminescent Substrate Liquid Storage Pool 22 and Washing Liquid Storage Pool 23 are respectively provided with openings communicating with the outside air; the liquid driving device is installed at Liquid Driving Force Inlet 13 to drive the flow of liquid in the chip; magnets (for example, Magnets 25a, 25b)are fixed outside Magnetic Beads-labeled Secondary Antibody Embedding Area 7, in order to fix Magnetic Beads 26. The magnetic-labeled secondary antibody embedding area is the liquid quantification chamber, which can be used to quantify the luminescent substrate liquid, and optionally, it can be further used to quantify the washing liquid.

A working mode of the micro-fluidic chip of this embodiment is as follows: a predetermined amount of liquid sample (such as serum or plasma diluted with a diluent) flows from Sample Inlet 2 through the main fluid channel to the Enzyme-labeled Primary Antibody Embedding Area 5 under the action of the liquid driving device, mixes and reacts with the enzyme-labeled primary antibody embedded inside, and then the reaction solution reaches Magnetic Beads-labeled Secondary Antibody Embedding Area 7continues to mix and react with the embedded magnetic-labeled secondary antibody inside. A reactant with a double antibody sandwich structure is formed on the magnetic beads. The magnetic beads are adsorbed by the magnet. The reactants are stabilized in Magnetic Beads-labeled Secondary Antibody Embedding Area 7 under the action of the magnetic beads, while the rest of the reaction liquid is discharged out of the chip through Liquid Driving Force Inlet 13under the action of the liquid driving device. Then, the air inflow port on the chip (such as the sample inlet) is closed, and Valve V3 between Washing Liquid Storage Pool 23 and Washing Liquid Inlet 12 is opened, and the washing liquid enters Magnetic Beads-labeled Secondary Antibody Embedding Area 7 through Washing Liquid Subchannel 18 under the action of the liquid driving device, to wash the magnetic beads inside. When the quantification of the washing liquid is completed in Magnetic Beads-labeled Secondary Antibody Embedding Area 7, Valve V3between Washing Liquid Storage Pool 23 and Washing Liquid Inlet 12 can be closed, the air inflow port is opened, the waste liquid is discharged from the chip through the Liquid Driving Force Inlet 13 under the action of the liquid driving device. In order to ensure the washing effect, it can be washed several times (the magnetic bead washing method is not limited to the method described here, the magnetic beads can also be washed by moving the magnet in the washing liquid); then the air inflow port (such as the sample inlet)on the chip is closed, and Valve V2 between Luminescent Substrate Liquid Storage Pool 22 and Luminescent Substrate Liquid Inlet 11 is opened, the luminescent substrate liquid enters Magnetic Beads-labeled Secondary Antibody Embedding Area 7 through Luminescent Substrate Liquid Subchannel 17 under the action of the liquid driving device, when the quantification of the luminescent substrate liquid is completed in Magnetic Beads-labeled Secondary Antibody Embedding Area 7, Valve V2 between Luminescent Substrate Liquid Storage Pool 22 and Luminescent Substrate Liquid Inlet 11 is closed, the liquid driving device stops driving, and the luminescent substrate liquid no longer flows into Magnetic Beads-labeled Secondary Antibody Embedding Area 7, and the air inflow port(such as the sample inlet)on the chip is opened, the quantified luminescent substrate liquid of the magnetic-labeled secondary antibody reacts with the reactant captured by the magnetic beads, and then the magnet is removed. The reaction liquid in Magnetic Beads-labeled Secondary Antibody Embedding Area 7 flows into Chemiluminescence Detection Area 9 under the action of the liquid driving device for detection.

The above-mentioned chemiluminescence micro-fluidic chip has a compact structure. For example, the magnetic-labeled secondary antibody embedding area is not only used to embed the magnetic-labeled secondary antibody, it can also be used as a liquid quantification chamber to quantify the luminescent substrate liquid, without the need to set up a additional liquid quantification chamber, and the magnetic-labeled secondary antibody embedding area can be further used as a magnetic bead washing area, with no need to set up a magnetic bead washing area, thus greatly saves the volume of the chip. Meanwhile, the reagent storage pool (such as luminescent substrate liquid storage pool, washing liquid storage pool, etc.) can be externally placed on the chip; comparing with that of reagent packs embedded in the chip in the prior art, the difficulty of the chip manufacturing process is reduced and the detection accuracy is improved.

It should be declared that the main fluid channel and multiple functional chambers can be formed inside the chip main body by various methods such as laser processing, mold injection processing, etc., and the top plate and the bottom plate can also be set separately,and particular structures can be made on the top plate or the bottom plate, and then the top plate or the bottom plate can be assembled together. Since the former processing method is comparably complicated, in a preferred embodiment, the chip main body includes Top Plate 1 and Bottom Plate 20; Top Plate 1 and Bottom Plate 20 are stacked and connected; at the connection place of Top Plate 1 and Bottom Plate 20 there provided with the main fluid channel and the multiple functional chambers; more preferably, Bottom Plate 20 is a smooth plate, and Top Plate 1is provided with micro pores, micro channels or micro cavities to form Sample Inlet 2, Liquid Driving Force Inlet 13, Luminescent Substrate Liquid Inlet 11, Washing Liquid Inlet 12, Luminescent Substrate Liquid Subchannel 17, Washing Liquid Subchannel 18, main fluid channel or multiple functional chambers, together with Bottom Plate 20. Such micro-fluidic chip is more convenient to prepare, and it further reduces the difficulty of the production process. Only the required particular structures on the top plate need to be processed, which further improves the production efficiency. In one embodiment, Bottom Plate 20 is a smooth flat plate, Top Plate 1 is provided with multiple micro channels on it to form a main fluid channel together with Bottom Plate 20; Top Plate 1 is provided with multiple micro cavities to form multiple functional chambers together with Bottom Plate 20; Top Plate 1 is provided with multiple holes to form Sample Inlet 2, Liquid Driving Force Inlet 13, Luminescent Substrate Liquid Inlet 11 and Washing Liquid Inlet 12together with Bottom Plate 20; to facilitate sampling, the size of Sample Inlet 2 is usually larger than the size of other inlets.

Therefore, the chip main body of the above-mentioned chemiluminescence micro-fluidic chip can include a top plate and a bottom plate that are stacked, and the structure that needs to be processed can be set on the top plate, and the bottom plate is only a smooth flat plate, thus the manufacturing difficulty can be further reduced, and the production efficiency is improved.

Optionally, a liquid identification site is also provided at the liquid inlet of the liquid quantification chamber. The setting of this liquid identification site can facilitate the monitoring and control of the liquid flow and possible bubbles in the chip. It can also realize the mixing of two liquids to be quantified, e.g. liquid samples and reagents (such as reaction reagents, sample processing reagents, etc.). Further, Enzyme-labeled Primary Antibody Embedding Area 5 is also a liquid quantification chamber. There provided Diluent Inlet 10 and Diluent Subchannel 16 on the chip main body; one end of Diluent Subchannel 16 communicates with Diluent Inlet 10, and the other end communicates with the liquid inlet of Enzyme-labeled Primary Antibody Embedding Area 5, and the sample diluent enters Enzyme-labeled Primary Antibody Embedding Area 5 through the diluent inlet and the diluent subchannel for quantification. Furthermore, the liquid inlet and liquid outlet of Enzyme-labeled Primary Antibody Embedding Area 5 are respectively provided with liquid identification sites, and the liquid to be quantified flows into Enzyme-labeled Primary Antibody Embedding Area 5 from its liquid inlet, and reaches the liquid outlet after filling Enzyme-labeled Primary Antibody Embedding Area 5. The sample diluent can not only dilute the liquid sample (such as serum, plasma, etc.), reduce its concentration and viscosity, and the substances contained in it can also reduce the background value of the liquid sample, making the detection more accurate, and the enzyme -labeled primary antibody can be better re-dissolved in sample diluents; in this technical solution, the enzyme-labeled primary antibody embedding area can be used to quantify the sample diluent without quantifying the sample diluent outside the chip. The quantified sample diluent can be mixed with a quantified liquid sample in the enzyme-labeled primary antibody embedding area, thus manpower could be saved and the operation is more convenient. When in use, Diluent Inlet 10 and Diluent Storage Pool 21 can be connected on and off by Valve V1. Diluent Storage Pool 21 is provided with an opening communicating with the outside air; a predetermined amount of liquid sample (such as serum or plasma) flows from Sample Inlet 2 through the main fluid channel to the liquid inlet of Enzyme-labeled Primary Antibody Embedding Area 5 under the action of the liquid driving device. Close the air inlet on the chip (such as the sample inlet), and open Diluent Storage Pool 21. Between Valve V1 and Diluent Inlet 10, the sample diluent enters Enzyme-labeled Primary Antibody Embedding Area 5 through Diluent Subchannel 16 under the action of the liquid driving device, and when it fills Enzyme-labeled Primary Antibody Embedding Area 5, and reaches the liquid outlet of Enzyme-labeled Primary Antibody Embedding Area 5, Valve V1 between Diluent Storage Pool 21 and Diluent Inlet 10 is closed, and the air inflow port (such as the sample inlet) is opened, and the liquid sample and the sample diluent can continue flow under the negative pressure of the liquid driving device, and they can be mixed in the main fluid channel and Enzyme-labeled Primary Antibody Embedding Area 5 under the positive and negative pressure of the liquid driving device. Of course, better mixing can also be realized by providing mixing channels.

Optionally, Chemiluminescence Detection Area 9 has a predetermined volume, and liquid identification sites are respectively provided at the liquid inlet and liquid outlet of Chemiluminescence Detection Area 9, and the liquid to be detected flows into Chemiluminescence Detection Area 9 through the liquid inlet of Chemiluminescence Detection Area 9, and reaches the liquid outlet after filling Chemiluminescence Detection Area 9; the volume of Chemiluminescence Detection Area 9 is less than or equal to the volume of Magnetic Beads-labeled Secondary Antibody Embedding Area 7. The liquid identification site provided at the liquid outlet of Chemiluminescence Detection Area 9 can be used to locate or fix the liquid identification device. When the reaction liquid after the luminescent substrate liquid reacts with the reactant captured by the magnetic beads reaches the liquid outlet of the chemiluminescence detection area, the liquid identification device sends out a signal, and the liquid driving device controls the reaction liquid to stop flowing, and the detection can be performed at this time.

Optionally, in order to facilitate the mixing of liquid samples and reagents (sample diluent, luminescent substrate liquid, etc.), the main fluid channel includes First Mixing Channel 6 and Second Mixing Channel 8; First Mixing Channel 6 is provided between Enzyme-labeled Primary Antibody Embedding Area 5 and Magnetic Beads-labeled Secondary Antibody Embedding Area 7; Second Mixing Channel 8 is provided between Magnetic Beads-labeled Secondary Antibody Embedding Area 7 and Chemiluminescence Detection Area 9.

Optionally, Sample Inlet 2 and Liquid Driving Force Inlet 13 are respectively provided at both ends of the main fluid channel.

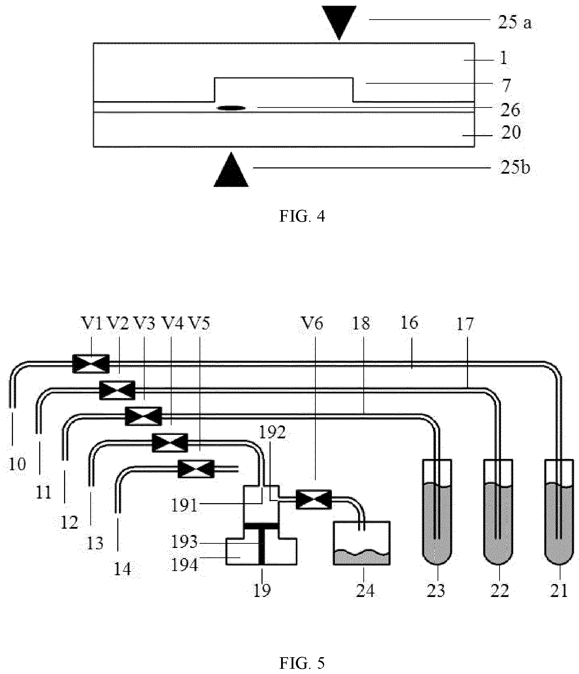

As shown in **FIG. 4****,** optionally, in order to facilitate the fixation of the magnetic beads, the chip main body and Magnetic Beads-labeled Secondary Antibody Embedding Area 7 are provided with magnet fixation sites at the corresponding positions; further, since the magnetic beads can be washed in Magnetic- Labeled Secondary Antibody Embedding Area 7, in order to better realize the washing of magnetic beads, two magnet fixation sites for locating Magnets 25a and 25b are arranged above and below Magnetic Beads-labeled Secondary Antibody Embedding Area 7, Magnets 25a and 25b correspond to the diagonal layout of Magnetic Beads-labeled Secondary Antibody Embedding Area 7.

Optionally, the liquid driving device is Plunger Pump 19, and the description of the plunger pump in Example 3 is applicable to this embodiment.

Optionally, functional chambers also include Sample Quantification Chamber 4. Sample Quantification Chamber 4 is also a liquid quantification chamber. The liquid sample flows into Sample Quantification Chamber 4 through the sample inlet for quantification; Sample Quantification Chamber 4 is located upstream of Enzyme-labeled Primary Antibody Embedding Area 5. On the micro-fluidic chip there provided Air Inlet 14 and Air Subchannel 27 communicating with the Air Inlet 14, and one end of Air Subchannel 27 communicates with Air Inlet 14, and the other end communicates with the main fluid channel between Sample Quantification Chamber 4 and Sample Inlet 2.The junction point of the other end of Air Subchannel 27 and the main fluid channel is adjacent to Sample Quantification Chamber 4. Here, "adjacent" can usually be understood as "1∼10mm from the liquid inlet of Sample Quantification Chamber 4". By providing the sample quantification chamber, the quantification of the liquid sample can be facilitated without additional quantification outside the chip, which makes the chip more convenient to use. Further, there is a liquid identification site at the liquid outlet of Sample Quantification Chamber 4, and the liquid to be quantified flows into Sample Quantification Chamber 4 from its liquid inlet, and reaches the liquid outlet after filling Sample Quantification Chamber 4. Furthermore, a liquid identification site is also provided at the liquid inlet of Sample Quantification Chamber 4.

When the micro-fluidic chip is used, the air inlet and the air pipe outside the chip can be connected on and off by a valve to control the air entering the chip. The liquid sample flows into the sample quantification chamber from the liquid inlet of the sample quantification chamber through the sample inlet under the action of the liquid driving device. When the liquid sample flows to the liquid outlet of the sample quantification chamber, the sample quantification chamber is filled. Then the liquid identification device located on the liquid identification site of the liquid outlet sends out an indication signal to control the air inlet to open. Because the air flow in the air subchannel requires a small driving pressure, and the flow of liquid samples requires a greater driving pressure, the liquid sample stays at the junction point of the air subchannel and the main fluid channel and does not continue to flow into the sample quantification chamber , and the quantification of the liquid sample can be realized in the sample quantification chamber. The quantified liquid sample can continue to flow to the enzyme-labeled primary antibody embedding area under the action of the liquid driving device.

Optionally, the liquid sample is whole blood. A Whole Blood Filtration Area 3 is provided between Sample Inlet 2 and Sample Quantification Chamber 4, and a whole blood filtration membrane is provided in Whole Blood Filtration Area 3; when the micro-fluidic chip is used for clinical diagnosis, whole blood is a common test sample. During testing, it is usually necessary to perform whole blood separation to separate the serum or plasma in the whole blood, and then react with the reagents; providing the whole blood filtration area in the chip is convenient for testing. Comparing with the method of quantifying whole blood in the first step and then separating the whole blood, providing a whole blood filtration area between the sample inlet and the sample quantification chamber can directly quantify the amount of serum or plasma in the sample quantification chamber, and the measurement result is more accurate . The material of the whole blood filtration membrane can be glass fiber,cotton linter fiber, polyester fiber, or blend fiber; optionally, the thickness of the whole blood filtration pad is 0.2-2.5mm; the adsorption speed of the whole blood filtration pad is 4-150 s/4cm, and the water absorption is 30-250 mg/cm².

The description of the liquid quantification chamber in Example 4 is applicable to the above-mentioned liquid quantification chamber (including Magnetic Beads-labeled Secondary Antibody Embedding Area 7, Enzyme-labeled Primary Antibody Embedding Area 5, and Sample Quantification Chamber 4), and will not be repeated here.

The description of the liquid identification site and the liquid identification device in Example 5 is applicable to the description of the liquid identification site and the liquid identification device described above, and will not be repeated here.

Optionally, the junction point of the other end of Luminescent Substrate Liquid Subchannel 17 and the liquid inlet of Magnetic Beads-labeled Secondary Antibody Embedding Area 7 is located on the main fluid channel of the liquid inlet of Magnetic Beads-labeled Secondary Antibody Embedding Area 7; in one embodiment, "adjacent"here is understood as "0.5∼10mm (preferably 0.5∼2mm) from the liquid inlet of Magnetic Beads-labeled Secondary Antibody Embedding Area 7".

Optionally, the washing liquid enters the Magnetic Beads-labeled Secondary Antibody Embedding Area 7 through Washing Liquid Inlet 12 and Washing Liquid Subchannel 18 for quantification; the junction point of the other end of Washing Liquid Subchannel 18 and the liquid inlet of Magnetic Beads-labeled Secondary Antibody Embedding Area 7 is located on the main fluid channel adjacent to the liquid inlet; in one embodiment, "adjacent" here is understood as "0.5∼10mm (preferably 0.5∼2mm) from the liquid inlet of Magnetic Beads-labeled Secondary Antibody Embedding Area 7". Preferably, the junction point of the other end of Washing Liquid Subchannel 18 and the liquid inlet of Magnetic Beads-labeled Secondary Antibody Embedding Area 7 is downstream of the junction point of the other end of Luminescent Substrate Liquid Subchannel 17 and the liquid inlet of Magnetic Beads-labeled Secondary Antibody Embedding Area 7, thus it can prevent the luminescent substrate liquid from being diluted by the washing liquid.

Optionally, the junction point of the other end of Diluent Subchannel 16 and the liquid inlet of Enzyme-labeled Primary Antibody Embedding Area 5 is located on the main fluid channel adjacent to the liquid inlet of Enzyme-labeled Primary Antibody Embedding Area 5; in one embodiment, "adjacent" here is understood as "0.5∼10mm (preferably 0.5∼2mm) from the liquid inlet of Enzyme-labeled Primary Antibody Embedding Area 5".

Optionally, the volume of Sample Inlet 2 is 5ul-300ul.

Optionally, the liquid outlet of Whole Blood Filtration Area 3 is a triangular liquid outlet; Whole Blood Filtration Area 3 has an area of 30-300mm², a width of 2-20mm, a length of 5-25mm, a depth of 0.3-3mm, and the angle of the front triangle is 15-160°.

Optionally, the volume of Sample Quantification Chamber 4 is 1-50ul.

Optionally, the volume of Enzyme-labeled Primary Antibody Embedding Area 5 is 5-50ul.

Optionally, the widths of First Mixing Channel 6 and Second Mixing Channel 8 are 200-2000um, the lengths are 5mm-40mm, and the depths are 0.2-3mm.

Optionally, the volume of Magnetic Beads-labeled Secondary Antibody Embedding Area 7 is 10-200ul.

Optionally, the volume of Chemiluminescence Detection Area 9 is 10-200ul.

Next, by referencing **FIGS. 1-5****,** a method for detecting a micro-fluidic chip according to an embodiment of the present invention will be described. The method includes Steps 101 to 110, and each step is specifically as follows:

Step 101: Insert the steel needles communicating separately with Diluent Storage Pool 21, Luminescent Substrate Liquid Storage Pool 22, Washing Liquid Storage Pool 23, Plunger Pump 19 and air into Sealing Gasket 15 in the chip, wherein the steel needles communicate separately with Diluent Inlet 10, Luminescent Substrate Liquid Inlet 11, Washing Liquid Inlet 12, Liquid Driving Force Inlet 13, Air Inlet 14; add the whole blood sample to Sample Inlet 2, open Electromagnetic Valve V4 and the negative pressure is generated by Plunger Pump 19 to transfer the whole blood sample into Whole Blood Filtration Area 3.

Step 102: The filtered serum of the whole blood sample is drawn into Sample Quantification Chamber 4, and the quantification of the serum is completed by Photoelectric Sensors (a1, a2) provided on the liquid inlet and liquid outlet of Sample Quantification Chamber 4.

When the whole blood sample passes over the Photoelectric Sensor a1, the output voltage value of the sensor changes, giving the system an identification signal to determine the liquid flow position in the chip. When the sample passes by Photoelectric Sensor a2, it is determined that Sample Quantification Chamber 4 is filled by the sample, and the inherent volume of this area is the quantitative value of the sample.

Step 103: Block Sample Inlet 2 and open Electromagnetic Valve V5, so that serum is drawn into Enzyme-labeled Primary Antibody Embedding Area 5.

Step 104: When Photoelectric Sensor (b1) provided on the liquid inlet of Enzyme-labeled Primary Antibody Embedding Area 5 detects serum, close Electromagnetic Valve V5 and open Electromagnetic Valve V1, so that the external sample diluent enters Enzyme-labeled Primary Antibody Embedding Area 5 from Electromagnetic Valve V1.

Step 105: When Photoelectric Sensor (b2) provided on the liquid outlet of Enzyme-labeled Primary Antibody Embedding Area 5 detects the external sample diluent, close Electromagnetic Valve V1, open Electromagnetic Valve V5, and successively generate positive pressure and negative pressure by Plunger Pump 19, and the serum, external diluent, and pre-embedded enzyme-labeled primary antibody flow back and forth to be re-dissolved between Enzyme-labeled Primary Antibody Embedding Area 5 and First Mixing Channel 6, to obtain the first mixture solution.

Step 106: The first mixture solution is drawn into Magnetic Beads-labeled Secondary Antibody Embedding Area 7, and is combined with the antigen antibody in Second Mixing Channel 8. The reactant formed is captured by the magnetic beads, and the magnetic beads are adsorbed by the magnet outside Magnetic Beads-labeled Secondary Antibody Embedding Area 7 and are stabilized in the Magnetic Beads-labeled Secondary Antibody Embedding Area 7. The rest of the reaction liquid is discharged from the chip by the liquid driving force inlet under the negative pressure of Plunger Pump 19, and then the next washing step is performed.

Step 107: Close Electromagnetic Valve V5 and open Electromagnetic Valve V3, so that the external washing liquid enters Magnetic Beads-labeled Secondary Antibody Embedding Area 7, and the injection volume of washing liquid is controlled by Photoelectric Sensors (c1, c2) provided on the liquid inlet and the liquid outlet of Magnetic Beads-labeled Secondary Antibody Embedding Area 7.

Step 108: After repeated washing the magnetic beads with the external washing liquid, Magnets 25a and 25b adsorb the magnetic beads, generate negative pressure by the plunger pump, and washed liquid is drawn to the external Waste Liquid Pool 24.

Step 109: Close Electromagnetic Valve V3, open Electromagnetic ValveV2, make the external luminescent substrate liquid enter Magnetic Beads-labeled Secondary Antibody Embedding Area 7, and the injection amount of the luminescent substrate liquid is controlled by Photoelectric Sensors (c1, c2).

Step 110: After the luminescent substrate liquid has fully reacted with the antigen and antibody on the magnetic beads, there obtained a reaction solution, and the reaction solution is transported to Chemiluminescence Detection Area 9 to perform the chemiluminescence detection; wherein, Photoelectric Sensors (d1, d2) provided on the liquid inlet and the liquid outlet of Chemiluminescence Detection Area 9 are used to detect the volume and position of the reaction solution.

The principle of the reaction between substances in the chemiluminescence micro-fluidic chip of this embodiment is the same as that of the magnetic particle immunochemiluminescence reaction, that is, the antigen in the sample combines the enzyme-labeled primary antibody (the primary antibody is labeled with HRP, AP and other catalytic groups), and combines the magnetic-labeled secondary antibody (the secondary antibody is fixed on the magnetic beads) to form a double-antibody sandwich complex, the magnetic beads are adsorbed by the magnet, the unbound antigen and enzyme-labeled primary antibody are washed away, and the substrate reaction liquid is added, the enzyme groups such as HRP and AP labeled on the primary antibody catalyze the substrate reaction liquid to emit light. The luminous intensity is proportional to the amount of antigen.

### Example 3

Refer to **FIG. 5****,** the present invention provides a liquid driving device that can realize the functions described in **Example1** or **Example2.** In this embodiment, the liquid driving device is Plunger Pump 19.

In terms of structure, the liquid driving device can be provided in a variety of types, such as existing injection pumps, diaphragm pumps, peristaltic pumps, etc. Anything that can drive the liquid to a predetermined area in the chip under pressure should fall into the protection scope of the present invention. Although injection pumps, diaphragm pumps, and peristaltic pumps can drive the liquid to flow, they cannot well control the liquid to stay in a specific position, and the plunger pump can better solve this problem. The plunger pump suitable for the present invention may be a plunger pump well known to those skilled in the art, which usually includes Pump Chamber 194 and Plunger 193. Pump Chamber 194 is provided with Liquid Inlet 191 and Liquid Outlet 192, and the top of Plunger 193 is inserted into the pump chamber, Plunger 193 reciprocates along the inner wall of Pump Chamber 194 in its axial direction; Liquid Inlet 191 and Liquid Outlet 192 are respectively provided with Valves V4 and V6. Since plunger pumps are mostly used for liquid drawn and discharge, the two openings provided on the pump chamber are usually called "the liquid inlet and the liquid outlet", but it should be noted that "the liquid inlet and the liquid outlet" here are not limited to liquid feeding and liquid drawn. In this embodiment, when the plunger pump is working, after Valve V4 at Liquid Inlet 191 is opened, the plunger moves downwards, and the pressure of the liquid near one end of Liquid Inlet191 of the plunger pump becomes smaller, resulting in a pressure difference between the two ends of the liquid, the liquid moves towards the direction of Liquid Inlet 191 under the action of the pressure difference. When the liquid reaches the predetermined position, the Valve V6 at the liquid outlet is opened to make the inside of the chip communicate with the outside air, and the pressure on both sides of the liquid is balanced under the action of air on both sides (the air on one side enters the chip through the the liquid outlet and the liquid inlet, and the air on the other side can flow into the opening(such as the sample inlet or an air passage provided separately) from the outside), and the liquid can stay at the predetermined position.

### Example 4

Refer to **FIG. 1** and **FIG. 3****,** the present invention provides a liquid quantification chamber that can realize the functions described in **Example1** or **Example2.**

It should be declared that, the liquid quantification chamber of the present invention can realize "the liquid to be quantified flows from the liquid inlet of the liquid quantification chamber into the liquid quantification chamber, and reaches the liquid outlet after filling the liquid quantification chamber", and its shape and structure can be selected according to actual needs, the present invention does not impose any limitation on this, for example, it can be a pipe shape, a polygonal shape, etc.

There are many ways to realize "the liquid to be quantified flows from the liquid inlet of the liquid quantification chamber into the liquid quantification chamber, and reaches the liquid outlet after filling the liquid quantification chamber", such as controlling the width and height of the liquid quantification chamber , carry out hydrophilic and hydrophobic treatment on the surface of the liquid quantification chamber, etc.

In this embodiment, the liquid quantification chamber is a chamber having a hexagonal structure. Optionally, the liquid inlet and the liquid outlet of the liquid quantification chamber are respectively two diagonal corners of the hexagonal structure; the angles of the two diagonal corners are less than 120°.

Optionally, the width of the liquid inlet of the liquid quantification chamber is 0.3-3mm (preferably 0.8-1.5mm) and the height is 0.3-3mm; the width of the liquid outlet of the liquid quantification chamber is 0.3-3mm (preferably 0.8-1.5mm), the height is 0.3-3mm. Both the liquid inlet width is too wide or too narrow and/or the height is too high or too low, is not conducive to the quantification. When the liquid inlet width is too wide or the height is too high, it is inclined to cause the liquid to flow to the liquid outlet of the liquid quantification chamber before the liquid quantification chamber is fully filled, thus it is impossible to achieve accurate liquid quantification. While the width of the liquid inlet is too narrow or the height is too low, the length needs to be increased to meet the volume requirements, which may lead to an increase in chip length and chip volume.

Optionally, the surface of the liquid quantification chamber is a surface formed by a hydrophilic surface modification; the width of the liquid inlet of the liquid quantification chamber is 0.3-5mm, and the height is 0.3-3mm; the width of the liquid outlet of the liquid quantification chamber is 0.3-5mm, the height is 0.3-3mm. The hydrophilic surface modification includes but not limited to plasmamodification, hydroxylation modification, carboxylation modification. After the surface of the liquid quantification chamber is hydrophilically modified, it is more conducive to the filling of the liquid in the cavity. By this time, the width of the liquid inlet and the liquid outlet of the liquid quantification chamber can be appropriately increased, thereby reducing the size of the liquid quantification chamber and the length of the chip.

Optionally, the surface of the liquid quantification chamber is a surface formed by hydrophobic surface modification. The width of the liquid inlet of the liquid quantification chamber is 0.3-2mm and the height is 0.3-3mm; the width of the liquid outlet of the liquid quantification chamber is 0.3-2mm, and the height is 0.3-3mm. Hydrophobic modification includes but not limited to hydrophobic physical modification and hydrophobic chemical modification (such as nanoparticle coating, chain alkyl group extending, etc.). After the surface of the liquid quantification chamber is modified to be a hydrophobic surface, the residual liquid on the inner wall can be prevented, thus ensures that the liquid reaches the liquid outlet after the liquid quantification chamber being filled.

### Example 5

Refer to **FIG. 2****,** the present invention provides a liquid identification site and a liquid identification device that can realize the functions described in **Example1** or **Example2.**

It should be declared that the liquid identification sites are used to locate or fix the liquid identification device. The present invention does not limit the structure of the liquid identification device, as long as the liquid identification function can be realized. For example, the liquid sensing device disclosed in the patent application with the publication number "CN105214744 A" can be used as the liquid identification device of the present invention, but the structure of such a liquid sensing device is relatively complicated, and the conductive needle needs to be built into the chip, and the conductive needle should contact with the reaction liquid, thus the experimental results is vulnerable to be effected under certain circumstances, and the preparation of the chip is comparably difficult. In the present embodiment, a more preferred liquid identification device is provided.

In this embodiment, the liquid identification site is used to locate the liquid identification device, the liquid identification device includes Light Source Generating Module 28 and Photoelectric Sensor 29; the liquid identification site includes the upper site for locating Light Source Generating Module 28 and the lower site for locating Photoelectric Sensor 29, the upper site and the lower site are respectively provided outside the chip main body; the upper site, the corresponding liquid inlet or liquid outlet, and the lower site are arranged sequentially in a vertical line. Correspondingly, Light Source Generating Module 28, the corresponding liquid inlet or liquid outlet and Photoelectric Sensor 29 are arranged sequentially in a vertical line. Since the liquid identification device can be provided at the liquid inlet or the liquid outlet of the liquid quantification chamber or at those of the test chamber, the "corresponding liquid inlet or liquid outlet" here corresponds to the liquid inlet or the liquid outlet of the liquid quantification chamber or those of the test chamber; for example, when the liquid outlet of the liquid quantification chamber is equipped with a liquid identification device, the light source generating module, the liquid outlet of the liquid quantification chamber and the Photoelectric Sensors are arranged successively in a vertical line; when the liquid inlet of the liquid quantification chamber is equipped with a liquid identification device, the light source generating module, the liquid inlet of the liquid quantification chamber and the Photoelectric Sensors are arranged successively in a vertical line; when the liquid outlet of the sample quantification chamber is equipped with a liquid identification device, the light source generating module, the liquid outlet of the sample quantification chamber and the Photoelectric Sensors are successively arranged in a vertical line.

Comparing with the conductive contact method, the optical sensing method for liquid identification, quantification and control reduces the interference of metal on the reaction system in the chip, which can improve the detection efficiency and thus the accuracy of quantification. Meanwhile, such identification device can be provided outside the micro-fluidic chip, that is, it is convenient to be fixed in the instrument instead of being provided on the chip, thus reducing the manufacturing difficulty of the chip. When using it, just align the light source generating module and Photoelectric Sensors with the liquid identification site. Specifically, the chip main body includes Top Plate 1 and Bottom Plate 20; Top Plate 1 and Bottom Plate 20 are stacked and connected; there provided the main fluid channels and multiple functional chambers at the connection place of Top Plate 1 and Bottom Plate 20; Light Source Generating Module 28 is located directly above the corresponding position of Top Plate 1 corresponding to the liquid inlet or the liquid outlet of the liquid quantification chamber, and Photoelectric Sensor 29 is located directly under the corresponding position of Bottom Plate 20 corresponding to the liquid inlet or the liquid outlet of the liquid quantification chamber.

The light source generating module is a module capable of providing light source, it can be LED, halogen lamp, laser lamp, etc. Under the illumination of the light source, due to the difference in light transmittance and refractive index of gas and liquid, the intensity of light irradiated to the Photoelectric Sensors is different, and thus the Photoelectric Sensors can identify between gas and liquid, thereby distinguishing whether the liquid reaches the sensing point. When the liquid flows to the liquid inlet or the liquid outlet, the liquid identification device can quickly identify it, thereby controlling the liquid driving device.

### Example 6

The embodiment of the present invention also provides an analytical instrument having a micro-fluidic chip, which includes an instrument frame, at least one reagent storage pool, a liquid driving device, a detection device, and the micro-fluidic chip in any of the above embodiments; wherein , the micro-fluidic chip is installed in the instrument frame; the liquid driving device is connected with the liquid driving force inlet of the micro-fluidic chip; the reagent storage pool can communicates on and off with the corresponding reagent inlet; the detection device is used to receive and process the detection signal from the micro-fluidic chip.

Optionally, the liquid driving device is a plunger pump; each of the reagent storage pools is provided with an opening communicating with the outside air.

The above are the preferred embodiments of the present invention. It should be pointed out that for those of ordinary skill in the art, several improvements and modifications can be made without departing from the principle of the present invention, and these improvements and modifications are also considered to be in the protection scope of the present invention.

## Claims

1. A micro-fluidic chip, comprising a chip main body, and a sample inlets, a liquid driving force inlets, a main fluid channel and multiple functional chambers provided on the chip main body;
The main fluid channel communicates with the multiple functional chambers, the sample inlet and the liquid driving force inlet respectively communicate with the main fluid channel, the liquid driving force inlet is used to connect a liquid driving device to drive liquid to flow in or out of functional chambers;
At least one of the multiple functional chambers is a liquid quantification chamber; the liquid quantification chamber has a predetermined volume, and a liquid identification site is provided at the liquid outlet of the liquid quantification chamber, and the liquid to be quantified flows into the liquid quantification chamber from the liquid inlet of the liquid quantification chamber, and reaches the liquid outlet after filling the liquid quantification chamber.

2. The micro-fluidic chip according to Claim 1, wherein the liquid quantification chamber comprises a reagent quantification chamber, the liquid inlet of the reagent quantification chamber communicates with one end of the reagent subchannel, and the other end of the reagent subchannel communicates with reagent inlet.

3. The micro-fluidic chip according to Claim 1, wherein a liquid identification site is also provided at the liquid inlet of the liquid quantification chamber.

4. The micro-fluidic chip according to Claim 1, wherein the liquid driving device is a plunger pump.

5. The micro-fluidic chip according to Claim 1, wherein the functional chamber comprises a test chamber, the test chamber has a predetermined volume, and a liquid identification site is provided at the liquid outlet of the test chamber, the liquid to be tested flows into the test chamber through the liquid inlet of the test chamber, and reaches the liquid outlet after filling the test chamber.

6. The micro-fluidic chip according to Claim 5, wherein a liquid identification site is also provided at the liquid inlet of the test chamber.

7. The micro-fluidic chip according to any one of Claims 1-6, wherein the liquid identification sites are used to locate a liquid identification device; the liquid identification device comprises a light source generating module and photoelectric sensors;
The liquid identification sites comprise an upper site for locating the light source generating module and a lower site for locating the photoelectric sensors, the upper site and the lower site are respectively located outside the chip main body; the positions of the upper site and the lower site correspond to the corresponding liquid outlet or liquid inlet, so that the positioned light source generating module, the corresponding liquid outlet or liquid inlet and the photoelectric sensors are successively arranged in a vertical line.

8. The micro-fluidic chip according to Claim 1, wherein the liquid quantification chamber is a hexagonal structure chamber.

9. The micro-fluidic chip according to Claim 1, wherein the liquid inlet of the liquid quantification chamber has a width of 0.3-3mm and a height of 0.3-3mm; the width of the liquid outlet of the liquid quantification chamber is 0.3-3mm, and the height is 0.3-3mm; or
The surface of the liquid quantification chamber is a surface formed by hydrophilic modification; the width of the liquid inlet of the liquid quantification chamber is 0.3-5mm, and the height is 0.3-3mm; the liquid outlet of the liquid quantification chamber The width is 0.3-5mm and the height is 0.3-3mm; or
The surface of the liquid quantification chamber is a surface formed by hydrophobic modification, the width of the liquid inlet of the liquid quantification chamber is 0.3-2mm, and the height is 0.3-3mm; the width of the liquid outlet of the liquid quantification chamber is 0.3-2mm and the height is 0.3-3mm.

10. The micro-fluidic chip according to Claim 1, wherein the chip main body comprises a top plate and a bottom plate; the top plate and the bottom plate are stacked and connected, and there provided the main fluid channel and the multiple functional chambers at the connection place of the top plate and the bottom plate.

11. The micro-fluidic chip according to Claim 10, wherein the bottom plate is a smooth flat plate, and the top plate is provided with micro pores, micro channels or micro cavities to form the sample inlet, the liquid driving force inlet, the main fluid channel or the functional chambers, together with the bottom plate.

12. The micro-fluidic chip according to Claim 1, wherein the sample inlet and the liquid driving force inlet are respectively arranged at both ends of the main fluid channel.

13. An analytical instrument having a micro-fluidic chip, **characterized by** comprising an instrument frame, at least one reagent storage pool, a liquid driving device, a detection device, and the micro-fluidic chip according to any one of Claims 1-12; Wherein, the micro-fluidic chip is installed in the instrument frame; the liquid driving device is connected to the liquid driving force inlet of the micro-fluidic chip; the reagent storage pool and the corresponding reagent inlet can be communicated on and off; the detection device is used for receiving and processing the detection signal sent by the micro-fluidic chip.

14. The analytical instrument having a micro-fluidic chip according to Claim 13, wherein the liquid driving device is a plunger pump; and each of the reagent storage pools is provided with an opening communicating with the outside air.
